(19) Europäisches Patentamt / European Patent Office / Office européen des brevets

(11) EP 3 816 157 B1

(12) **EUROPEAN PATENT SPECIFICATION**

(45) Date of publication and mention of the grant of the patent:
**13.07.2022 Bulletin 2022/28**

(21) Application number: **20203996.2**

(22) Date of filing: **27.10.2020**

(51) International Patent Classification (IPC):
***C07D 233/52*** *(2006.01)* ***A61K 31/4168*** *(2006.01)*
***A61P 35/00*** *(2006.01)*

(52) Cooperative Patent Classification (CPC):
**C07D 233/52; A61P 35/00**

(54) **9-BENZENESULFONIC ACID-10-IMIDAZOLYLANTHRAHYDRAZONE AND SYNTHESIS METHOD AND APPLICATION THEREOF**

9-BENZOLSULFONSÄURE-10-IMIDAZOLYLANTHRAHYDRAZON SOWIE SYNTHESEVERFAHREN UND ANWENDUNG DAVON

ACIDE 9-BENZÈNESULFONIQUE-10-IMIDAZOLYLANTHRAHYDRAZONE ET PROCÉDÉ DE SYNTHÈSE ET APPLICATION ASSOCIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.10.2019 CN 201911029635**

(43) Date of publication of application:
**05.05.2021 Bulletin 2021/18**

(73) Proprietor: **Guangxi Normal University**
**Guilin City, Guangxi 541004 (CN)**

(72) Inventors:
- **Liu, Yancheng**
  **Guilin, Guangxi province 541004 (CN)**
- **Liang, Hong**
  **Guilin City, Guangxi 541004 (CN)**
- **Chen, Zhenfeng**
  **Guilin City, Guangxi 541004 (CN)**
- **Liu, Ruixue**
  **Guilin City, Guangxi 541004 (CN)**
- **Wu, Yingshu**
  **Guilin City, Guangxi 541004 (CN)**
- **Tang, Mengting**
  **Guilin, Guangxi province 541004 (CN)**

(74) Representative: **Gille Hrabal**
**Partnerschaftsgesellschaft mbB**
**Patentanwälte**
**Brucknerstraße 20**
**40593 Düsseldorf (DE)**

(56) References cited:
- **WUNZ T P ET AL: "NEW ANTITUMOR AGENTS CONTAINING THE ANTHRACENE NUCLEUS", JOURNAL OF MEDICINAL CHEMISTRY, AMERICAN CHEMICAL SOCIETY, US, vol. 30, 1 January 1987 (1987-01-01), pages 1313-1321, XP000867863, ISSN: 0022-2623, DOI: 10.1021/JM00391A009**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 816 157 B1

## Description

### TECHNICAL FIELD

The invention relates to 10-imidazolylanthrahydrazone-9-imino benzenesulfonate sodium and synthesis method and applications thereof, and it is in the medical technical field.

### BACKGROUND ART

Anthracycline anticancer medicines generally refer to a series of natural antitumor antibiotics and derivatives thereof with anthraquinone as the structural basis, such as Daunorubicin, Doxorubicin, Mitoxantrone, and the like. At present, this kind of antitumor antibiotics are successfully applied in clinical antitumor treatments and they are remarkable in the curative effect. Meanwhile, the compounds play an important role in the combined chemotherapy scheme of CEF, AC and CAF and have remarkable curative effects, especially on leukemia, breast cancer, ovarian cancer, lung cancer and the like. In recent years, novel non-anthraquinone anthracycline anticancer medicines have been synthesized and successfully developed, and for example, Bisantrene just is a typical example. The anthrylhydrazone side chain group of the Bisantrene is a special 5-membered imidazoline group, and when an anthracene ring is inserted between DNA base pairs, the side chain group can further stabilize this combination through hydrogen bonds and electrostatic interactions.

The inventors team of the application has reported in the invention patent with the publication number CN103086975A as previously filed that the 9-formyl-10-imidazolylanthrahydrazone having a structure of the following formula (II) is obtained by aldehyde-amine condensation reaction between 9,10-anthracene dicarboxaldehyde and 2-hydrazino-2-imidazoline hydrobromide and then by purification with silica gel:

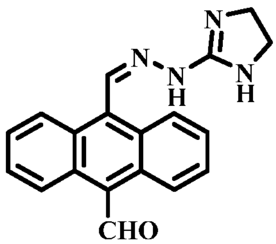

(II).

In subsequent researches, the inventors of the application find that the 9-formyl-10-imidazolylanthrahydrazone has certain biological activities, but it is insoluble in water, which limits further applications of the compound. At present, there is no report about hydrophilic modifications of the 9-formyl-10-imidazolylanthrahydrazone with benzenesulfonic acid groups in order to improve the hydrophilicity of the obtained compound whilst increasing the activities thereof.

### SUMMARY OF THE INVENTION

The technical problem to be solved by the invention is to provide a 10-imidazolylanthrahydrazone-9-imino benzenesulfonate sodium that, as compared to its precursor compound 9-formyl-10- imidazolylanthrahydrazone, is improved both in the water-solubility and the biological activity through modifications with benzenesulfonic acid groups, and a synthesis method and application thereof.

The 10-imidazolylanthrahydrazone-9-imino benzenesulfonate sodium according to the invention is a compound represented by the formula (I) or a pharmaceutically acceptable salt thereof:

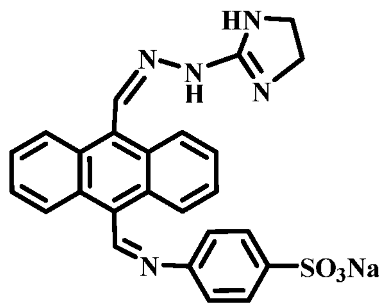

(I).

The invention uses a benzenesulfonic acid group to modify the 9-position of the 9-formyl-10-imidazolylanthrahydrazone which can be improved in the bioavailability by the virtue of the introduction of the benzenesulfonic acid group. That is, at the 9-postion, through an acetal reaction, the benzenesulfonic acid group linked to the C=N imine bond is formed, and it becomes a control of the C=N-N hydrazone bond at the 10-position. By utilizing the characteristics of the imine bond having higher reaction activities than the hydrazone bond and exhibiting acidic sensibility, the obtained compound, in a slightly acidic micro-environment of malignant tumor, is more easily broken to release an active anthrylhydrazone compound, thereby to act the targeting function on malignant tumors in a molecular level.

The invention further provides a synthesis method for 10-imidazolylanthrahydrazone-9-imino benzenesulfonate sodium, particularly comprising the following steps: placing 9-formyl-10-imidazolylanthrahydrazone and sodium p-aminobenzenesulfonate in an alcohol solvent in the presence or absence of a catalyst, and reacting them at heating conditions, to obtain the target compound.

In the synthesis method according to the invention, the raw material 9-formyl-10-imidazolylanthrahydrazone is prepared according to the invention patent with the publication number CN103086975A, and no detail is further provided here.

In the synthesis method according to the invention, the molar ratio of 9-formyl-10-imidazolylanthrahydrazone to sodium p-aminobenzenesulfonate is a stoichiometric ratio, and during actual operations, the sodium p-aminobenzenesulfonate may be slightly excessive. Typically, the molar ratio of sodium p-aminobenzenesulfonate to 9-formyl-10-imidazolylanthrahydrazone is 1 to 1.2: 1.

In the synthesis method according to the invention, the alcohol solvent may be any water-free alcohol solvent, more preferably, which is dehydrated with a molecular sieve before use. The alcohol solvent may be selected from alcohols containing 1 to 3 carbon atoms or combinations of more than two of them, particularly, selected from methanol, ethanol, n-propanol and isopropanol or combinations of more than two of them. The alcohol solvent is typically used in an amount that the solvent can dissolve all raw materials participating the reaction, and particularly, with calculations based on from 0.10 to 0.30 mmol of 9-formyl-10-imidazolylanthrahydrazone, the total amount of the alcohol solvent required by dissolving all raw materials is typically in the range of from 10 to 30 mL.

In the synthesis method according to the invention, the addition of a catalyst can effectively increase the reaction rate. The catalyst is p-methylbenzenesulfonic acid, and the amount of the catalyst is usually 0.5-1.5 times the molar amount of the 9-formyl-10-imidazolylanthrahydrazone.

In the synthesis method according to the invention, the reaction is preferably performed at temperatures of more than or equal to 40 °C, more preferably at temperatures from 50 to 80 °C. When the reaction is performed at temperatures from 40 to 80 °C in the presence of a catalyst, it is necessary to typically control the reaction time in the range from 2 to 10 h, and in the absence of a catalyst, it is necessary to typically control the reaction time to be more than 5 h.

The invention further includes use of the above 10-imidazolylanthrahydrazone-9-imino benzenesulfonate sodium or a pharmaceutically accept salt thereof in manufacture of antitumor medicines.

The invention further includes a pharmaceutical composition, comprising a therapeutically effective amount of 10-imidazolylanthrahydrazone-9-imino benzenesulfonate sodium or a pharmaceutically acceptable salt thereof.

As compared to the prior art techniques, the invention, through an imine bond and a hydrazone bond, respectively introduce an imidazoline group and a benzenesulfonic acid group at the 10-position and 9-position of the 9-formyl-10-imidazolylanthrahydrazone, to obtain an asymmetric anthrylhydrazone with a novel structure, 10-imidazolylanthrahydrazone-9-imino benzenesulfonate sodium, and as compared to the 9-formyl-10-imidazolylanthrahydrazone, the 10-imidazolylanthrahydrazone-9-imino benzenesulfonate sodium according to the invention are remarkably improved in both the water solubility and the biological activity.

## ILLUSTRATIONS TO THE DRAWINGS OF THE DESCRIPTION

FIG. 1 is an infrared (IR) spectrum of the final product prepared in Example 1 of the invention.

FIG. 2 is a hydrogen nuclear magnetic resonance (NMR) spectrum of the final product prepared in Example 1 of the invention.

FIG. 3 is an electro-spray ionization mass spectrum (EM) of the final product prepared in Example 1 of the invention.

**BEST MODES FOR CARRYING OUT THE INVENTION**

The following specific examples are combined to further illustrate the invention so as to better understand the contents in the invention. However, the invention is not limited to the following examples.

The 9-formyl-10-imidazolylanthrahydrazone described in each of the following examples is prepared according to the following method:

1) 10 mmol of 9,10-anthracene dicarboxaldehyde and 10 mmol of 2-hydrazino-2-imidazoline hydrobromide were dissolved in 70 mL of ethanol and reacted under reflux conditions at 78 °C. Through tracks and detections with a thin layer chromatography (TLC), upon completion of the reaction (about 8 h), the reaction was stopped and the reaction solution was filtered whilst it was still hot; the resultant solid was filtered in vacuum dried for 10 h, to obtain a mixed product with the yield of 85%.

2) The resultant mixed product was passed through a silica gel chromatographic column and eluted by a mixed eluting agent consisting of methanol and methane dichloride in a volume ratio of 1:30 to 80, and through tracks and detections with a thin layer chromatography, an eluting solution containing the target ingredient was collected. The resultant eluting solution was concentrated and dried to produce a red solid product, with the yield of 60%.

The resultant red solid product was respectively identified by IR spectrum, elemental analyses, $^1$H NMR spectrum, and electrospray mass spectrum, and the specific spectrum characteristic data were shown below:

IR Spectrum: (KBr, $cm^{-1}$) 3432 ($v_{N-H}$), 1661 ($v_{C=N}$).
Elemental Analysis: Calcd. C, 72.13; H, 5.10; N, 17.71%; Found: C, 72.16; H, 5.16; N, 17.67%.
NMR Spectrum (hydrogen) : $^1$H NMR (500 MHz, DMSO-$d_6$) $\delta$11.46 (s, 1H), 9.16 (s, 1H), 9.00 (d,*J*=8.9 Hz, 2H), 8.68 (d,*J* = 8.8 Hz, 2H), 7.76-7.70 (m, 2H), 7.67-7.60 (m, 2H), 6.83 (d,*J*=15.4 Hz, 2H), 3.45 (s, 4H).
Cationic peak of electro-spray ionization mass spectrum: m/z=317.14$[L+H]^+$ (L represents 9-formyl-10-imidazolylanthrahydrazone ).

Hence, the above red solid product was determined to be 9-formyl-10-imidazolylanthrahydrazone, with the molecular formula $C_{19}H_{16}N_4O$, the molecular weight 316 g/mol, and a structure represented by the following formula(II):

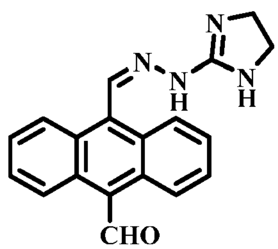

(II).

**Example 1**

0.15 mmol of 9-formyl-10-imidazolylanthrahydrazone, 0.15 mmol of sodium p-aminobenzenesulfonate, and 0.15 mmol of p-methylbenzenesulfonic acid were dissolved in 20.0 mL of methanol, and the resultant mixed solution was reacted at 65 °C (condensed reflux) for 8 hours. The resultant reaction material was cooled to generate a large quantity of orange solids, and it was filtered to collect the filtering cake, thereby to obtain an orange powder solid product. The solid powder was purified by recrystallization with methanol, to produce orange microcrystalline solids, with the yield of 50%.

The above orange microcrystalline solid product was respectively identified by IR spectrum, elemental analyses, $^1$H NMR spectrum, and electrospray mass spectrum, and the specific spectra were shown in FIG. 1, FIG. 2 and FIG.3 whilst the specific spectrum characteristic data were shown below:

IR spectrum: (KBr, $cm^{-1}$) 3415 ($v_{N-H}$), 1665 ($v_{C=N}$); 1197, 1045 (vs=o).

NMR Spectrum (hydrogen): $^1H$ NMR (400 MHz, DMSO) 512.55 (s, 1H), 9.72 (s, 1H), 9.34 (s, 1H), 8.77-8.73 (m, 2H), 8.48-8.43 (m, 2H), 7.77-7.73 (m, 2H), 7.69 (qd, J=6.3, 3.3 Hz, 4H), 7.48-7.44 (m, 2H), 3.76 (s, 4H).

Anionc peak of Electrospray Mass Spectrum: m/z=470.12$[L-Na]^-$ (L represents 9-formyl-10-imidazolylanthrahydrazone ).

Elemental Analysis: Calcd. C, 60.84; H, 4.08; N, 14.19%; Found: C, 60.80; H, 4.12; N, 14.17%.

Hence, the above orange product was determined to be 10-imidazolylanthrahydrazone-9-imino benzenesulfonate sodium, with the molecular formula $C_{25}H_{20}N_5NaO_3S$, the molecular weight 493.11 g/mol, and a structure represented by the following formula(I):

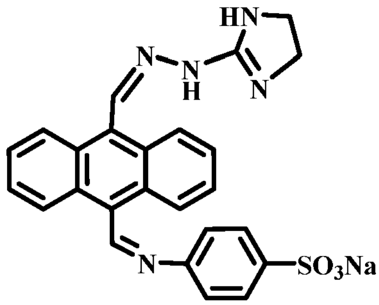

(I).

**Example 2**

0.25 mmol of 9-formyl-10-imidazolylanthrahydrazone and 0.25 mmol of sodium p-aminobenzenesulfonate were dissolved in 18 mL of ethanol, and the resultant mixed solution was reacted at 78 °C (condensed reflux) for 6 hours. After the reaction, the solution was cooled to obtain an orange suspension which was filtered to collect the filtering cake, thereby to obtain an orange powder solid, with the yield of 45%.

The product obtained in the example was respectively identified by IR spectrum, elemental analyses, $^1H$ NMR spectrum, and electrospray mass spectrum, to confirm that the resultant orange solid powder was the target product, 10-imidazolylanthrahydrazone-9-imino benzenesulfonate sodium .

**Example 3**

0.20 mmol of 9-formyl-10-imidazolylanthrahydrazone and 0.25 mmol of sodium p-aminobenzenesulfonate were dissolved in 10 mL of methanol and 5 mL of ethanol, and the resultant mixed solution was reacted at 60 °C (condensed reflux) for 6 hours. After the reaction, the solution was cooled to obtain an orange suspension which was filtered to collect the filtering cake, thereby to obtain an orange solid powder, with the yield of 40%.

The product obtained in the example was respectively identified by IR spectrum, elemental analyses, $^1H$ NMR spectrum, and electrospray mass spectrum, to confirm that the resultant orange solid powder was the target product, 10-imidazolylanthrahydrazone-9-imino benzenesulfonate sodium .

**Example 4**

The example was performed by repeating Example 1, differing only in that the methanol was replaced by isopropanol, and the reaction was performed at 80 °C for 2 hours.

As a result, an orange solid powder was obtained, with the yield of 40%.

The product obtained in the example was respectively identified by IR spectrum, elemental analyses, 1H NMR spectrum, and electrospray mass spectrum, to confirm that the resultant orange solid powder was the target product, 10-imidazolylanthrahydrazone-9-imino benzenesulfonate sodium .

**Example 5**

The example was performed by repeating Example 1, differing only in that the reaction was performed at 40 °C.

As a result, an orange solid powder was obtained, with the yield of 40%.

The product obtained in the example was respectively identified by IR spectrum, elemental analyses, 1H NMR spectrum, and electrospray mass spectrum, to confirm that the resultant orange solid powder was the target product,

10-imidazolylanthrahydrazone-9-imino benzenesulfonate sodium .

**Test 1: Solubility Test**

At 25 °C, 9-formyl-10-imidazolylanthrahydrazone (the raw material used in Example 1 of the invention), 10-imidazolylanthrahydrazone-9-imino benzenesulfonate sodium (the target product prepared in Example 1 of the invention), and 9-hydroxymethyl-10-imidazolylanthrahydrazone (prepared by referring to the invention patent with the publication number CN103086975A) were respectively dissolved in 100g of water, to measure the solubility.

The results were shown below:

9-formyl-10-imidazolylanthrahydrazone, water-insoluble at 25°C;
10-imidazolylanthrahydrazone-9-imino benzenesulfonate sodium, having a solubility in water, the solubility at 25 °C: 0.155 g/100 g $H_2O$;
9-hydroxymethyl-10-imidazolylanthrahydrazone, having a solubility in water, the solubility at 25 °C: 0.095 g/100 g $H_2O$.

**Test 2: In Vitro Test of Antitumor Activity**

**1. Culture of Cell Lines**

The test selected human ovarian cancer cell SK-OV-3, liver cancer cell Hep-G2, gastric cancer cell MGC-803, bladder cancer cell T-24, cervical cancer cell HeLa-229, human large cell lung cancer cell NCI-H460 and human normal liver cell HL-7702, and all the used cell lines were placed in an RPMI-1640 or high-sugar DMEM culture solution containing 1% penicillin-streptomycin and 10% newborn fetal calf serum and cultured in an incubator at 37 °C and 5% $CO_2$. The growths of the cells were observed discontinuously by an inverted microscope, and the culture solution was replaced periodically or digestion subcultured with 0.25% trypsin according to actual situations.

**2. Preliminary Screening of Activity**

The invention used the medicines 9-formyl-10-imidazolylanthrahydrazone (the raw material used in Example 1 of the invention), and 10-imidazolylanthrahydrazone-9-imino benzenesulfonate sodium (prepared by the method described in Example 1 of the invention), each having a purity of ≥95%, and cisplatin was used as a control. The compounds each were formulated into 20 μmol/L of a solution, DMSO is used as a cosolvent with a final concentration of ≤1%; at the concentration, the inhibition degrees of the compounds against the growth of tumor cells were tested.

**3. Inhibition Test Against Cell Growth**

(1) After the compounds were dissolved in the presence of the cosolvent DMSO (a DMSO final concentration of ≤1%) and diluted by using corresponding culture media to form working solutions having final concentration of 20 μmol/L and stored at 4 °C.
(2) Subsequently, different tumor cells in the logarithmic growth phase were uniformly inoculated on a 96-well plate (the hole sites at the edge of the plate were filled with bacteria-free PBS) respectively. After the individual tumor cells were attached to the wall, the working solutions of the tested compounds were added, and each compound is provided with quintuple holes in parallel whilst a corresponding control group (with a culture solution only containing tumor cells and the same equivalent of DMSO but without the tested compounds) is provided. At equivalent conditions, the cultures of the cells were continued for 48 hours.
(3) At 4 to 6 hours before completion of the cultures, 10 μL of MTT (5 mg/mL PBS, i.e., 0.5% MTT) were added to each pole, and the culture was continued for 4 to 6 hours.
(4) After terminating the cultures, the residual culture solution in the poles was carefully aspirated off, and each pore was added with 100 μl of DMSO and shaken by a flat-plate oscillator for 10 min so as to fully dissolve formazane crystals. Then, the blank control group was zero set, and an enzyme-labeling instrument was used to measure the absorbance (Å) of each pore at a dual wavelength of 570 nm/630 nm, thereby to measure the inhibition rate.
(5) According to the measured optical densities (OD value), the quantity of active cells were evaluated, i.e., the higher the OD value, the stronger the cell activities. The following equitation was utilized:

**Inhibition rate against growth of tumor cells (%) = 100%×(1－average OD value of experimental group/average OD of control group).**

According to the results of preliminary screening inhibition rates, different concentration gradients are further set to measure inhibition rates at different concentrations, and further to calculate the half inhibition concentrations (ICso value) of the compounds, and the results are shown in Table 3 below:

Table 3 the ICso (μM) of the tested medicines against different cell lines

| | 9-formyl-10-imidazolylanthrahydrazone | 10-imidazolylanthrahydrazone-9-imino benzenesulfonate sodium | cisplatin |
|---|---|---|---|
| T-24 | 13.88±1.46 | 10.65±0.56 | 14.79±2.74 |
| HepG-2 | 15.12±1.69 | 12.71±1.56 | 18.55±0.78 |
| MGC-803 | 18.82±0.50 | 9.48±0.68 | 17.79±2.50 |
| HeLa-229 | >20 | 10.45±1.52 | 13.21±2.74 |
| NCI-H460 | 16.14±1.40 | 14.54±1.42 | 18.29±1.02 |
| HL-7702 | 16.40±2.78 | 11.34±0.71 | 12.67±1.27 |

As seen from the test results of antitumor activities, 9-formyl-10-imidazolylanthrahydrazone and 10-imidazolylanthrahydrazone-9-imino benzenesulfonate sodium have remarkable proliferation inhibition activities against most tumor cell lines, and the inhibition activities, in general, are superior to the positive control medicine, cisplatin, in the control test.

Regarding the 9-formyl-10-imidazolylanthrahydrazone, it exhibits good growth inhibitions against the tumor cells except for human cervical cancer cells HeLa-229 against which the compound exhibits weak activity, wherein the inhibition against human bladder cancer cells T-24 is relatively strong, with $IC_{50}$ value of 13.88±1.46 μM. The target product 10-imidazolylanthrahydrazone-9-imino benzenesulfonate sodium of the invention has more remarkable proliferation inhibition activities against all the tested tumor cells, wherein the inhibitions against human gastric cancer cells MGC-803 and human cervical cancer cells HeLa-229 are strongest, with the $IC_{50}$ values of 9.48±0.68 μM and 10.45±1.52 μM, respectively, and as compared to the 9-formyl-10-imidazolylanthrahydrazone, its activities are remarkably increased by more than two times.

To sum up, the10-imidazolylanthrahydrazone-9-imino benzenesulfonate sodium of the invention exhibits good antitumor activities in vitro, and its prominent proliferation inhibition activities against a variety of typical tumor cells are manifested as that after the benzenesulfonic acid is introduced into the 9-position, the antitumor activities of the anthrylhydrazone are effectively improved. Clearly, the compound has good potential medicinal values, and it is worthy of being deeply developed and expectable to be used in manufacture of various antitumor medicines.

## Claims

1. A compound of formula (I) or a pharmaceutically acceptable salt thereof:

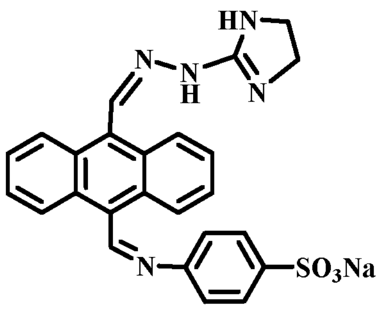

(I).

2. A synthesis method for a compound as claimed in claim 1, **characterized in that** it comprises the steps: placing 9-formyl-10-imidazolylanthrahydrazone and sodium p-aminobenzenesulfonate in an alcohol solvent in the presence

or absence of a catalyst, and reacting them at heating conditions, to obtain a target compound.

3. The synthesis method as claimed in claim 2, **characterized in that**: the catalyst is p-methylbenzenesulfonic acid.

4. The synthesis method as claimed in claim 2, **characterized in that**: the alcohol solvent is selected from one of alcohols containing 1 to 3 carbon atoms or combinations of more than two of them.

5. The synthesis method as claimed in claim 2, **characterized in that**: the reaction is performed at temperatures of more than or equal to 40 °C.

6. The synthesis method as claimed in claim 5, **characterized in that**: the reaction is performed at temperatures of 50 to 80 °C.

7. Use of a compound or a pharmaceutically acceptable salt thereof as claimed in claim 1 in manufacture of antitumor medicines.

8. A pharmaceutical composition, comprising a therapeutically effective amount of a compound or a pharmaceutically acceptable salt thereof as claimed in claim 1.

**Patentansprüche**

1. Eine Verbindung der Formel (I) oder ein pharmazeutisch annehmbares Salz davon:

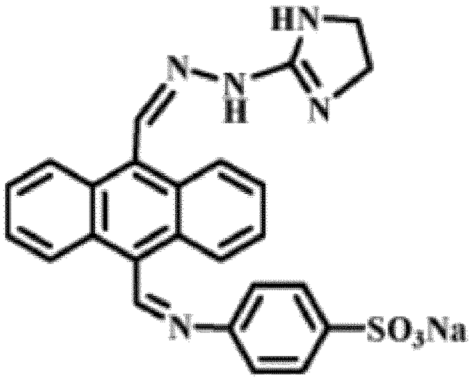

(I).

2. Syntheseverfahren für eine Verbindung nach Anspruch 1, **dadurch gekennzeichnet, daß** es die folgenden Schritte umfaßt: Einbringen von 9-Formyl-10-imidazolylanthrahydrazon und Natrium-p-aminobenzolsulfonat in ein alkoholisches Lösungsmittel in Gegenwart oder Abwesenheit eines Katalysators, und deren Umsetzung unter Erwärmung, um eine Zielverbindung zu erhalten.

3. Syntheseverfahren nach Anspruch 2, **dadurch gekennzeichnet, dass**: der Katalysator p-Methylbenzolsulfonsäure ist.

4. Syntheseverfahren nach Anspruch 2, **dadurch gekennzeichnet, dass**: das alkoholische Lösungsmittel ausgewählt ist aus einem der Alkohole mit 1 bis 3 Kohlenstoffatomen oder Kombinationen von mehr als zwei davon.

5. Syntheseverfahren nach Anspruch 2, **dadurch gekennzeichnet, dass**: die Reaktion bei Temperaturen von 40 °C oder darüber durchgeführt wird.

6. Syntheseverfahren nach Anspruch 5, **dadurch gekennzeichnet, daß**: die Reaktion bei Temperaturen bei Temperaturen von 50 bis 80 °C durchgeführt wird.

7. Verwendung einer Verbindung oder eines pharmazeutisch annehmbaren Salzes davon nach Anspruch 1 bei der Herstellung von Antitumor-Medikamenten.

8. Pharmazeutische Zusammensetzung, umfassend eine therapeutisch wirksame Menge einer Verbindung oder eines pharmazeutisch annehmbaren Salzes davon gemäß Anspruch 1.

## Revendications

1. Un composé de formule (I) ou un de ses sels pharmaceutiquement acceptables:

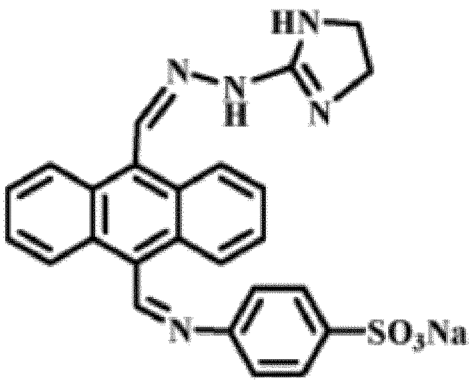

(I).

2. Procédé de synthèse d'un composé selon la revendication 1, **caractérisé en ce qu'**il comprend les étapes suivantes: l'introduction de 9-formyl-10-imidazolylanthrahydrazone et de p-aminobenzènesulfonate de sodium dans un solvant alcoolique en présence ou en l'absence d'un catalyseur, et leur réaction sous chauffage pour obtenir un composé cible.

3. Procédé de synthèse selon la revendication 2, **caractérisé en ce que**: le catalyseur est l'acide p-méthylbenzènesulfonique.

4. Procédé de synthèse selon la revendication 2, **caractérisé en ce que**: le solvant alcoolique est choisi parmi l'un des alcools ayant de 1 à 3 atomes de carbone ou des combinaisons de plus de deux d'entre eux.

5. Procédé de synthèse selon la revendication 2, **caractérisé en ce que**: la réaction est effectuée à des températures égales ou supérieures à 40°C.

6. Procédé de synthèse selon la revendication 5, **caractérisé en ce que**: la réaction est effectuée à des températures comprises entre 50 et 80 °C.

7. Utilisation d'un composé ou d'un sel pharmaceutiquement acceptable de celui-ci selon la revendication 1 dans la préparation de médicaments antitumoraux.

8. Composition pharmaceutique comprenant une quantité thérapeutiquement efficace d'un composé ou d'un sel pharmaceutiquement acceptable de celui-ci selon la revendication 1.

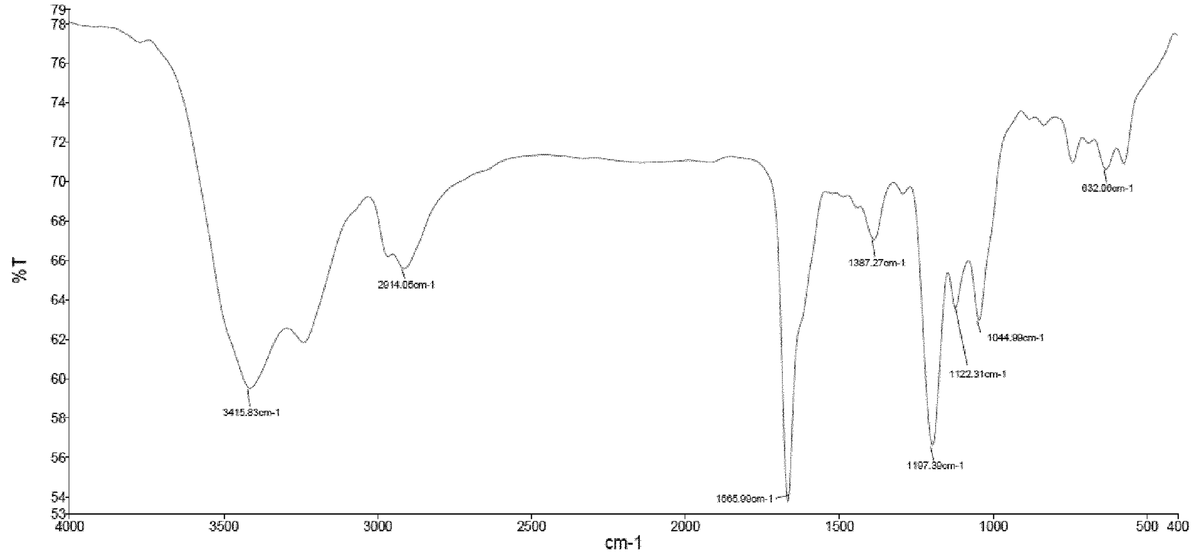


**Figure 1**

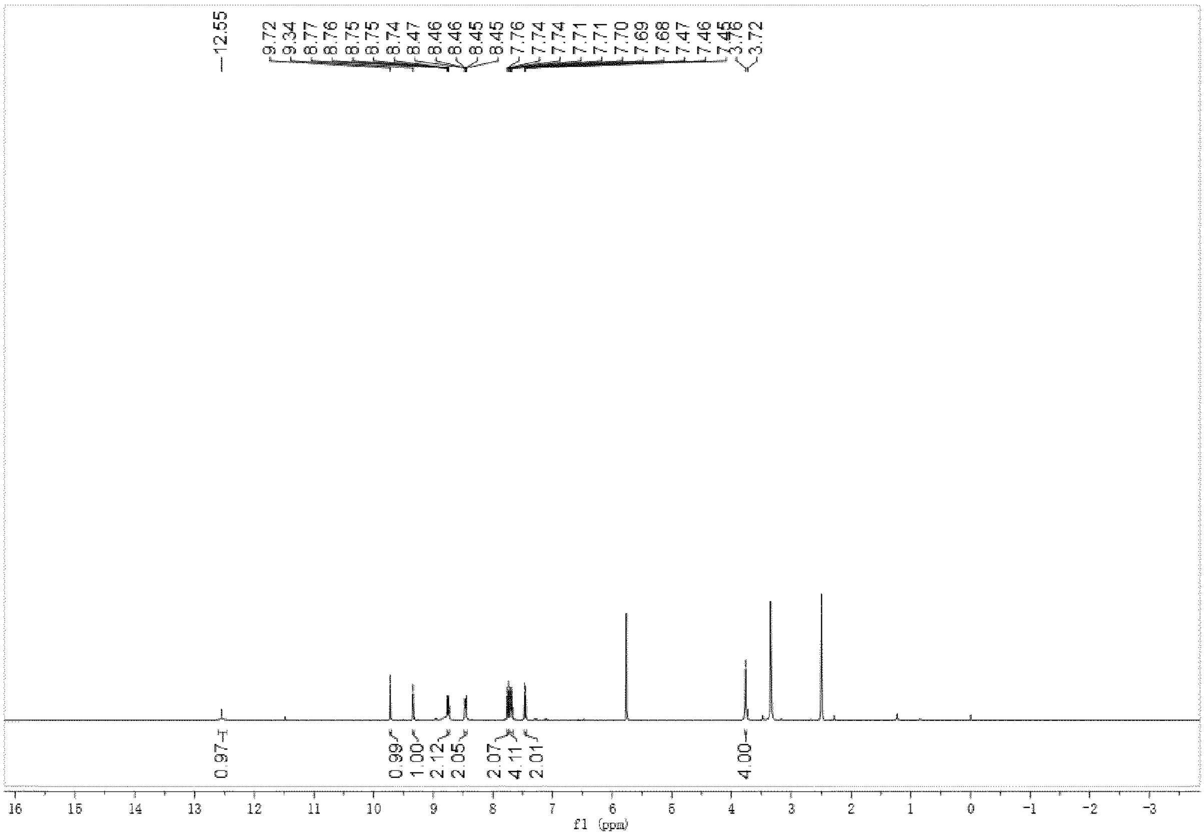


**Figure 2**

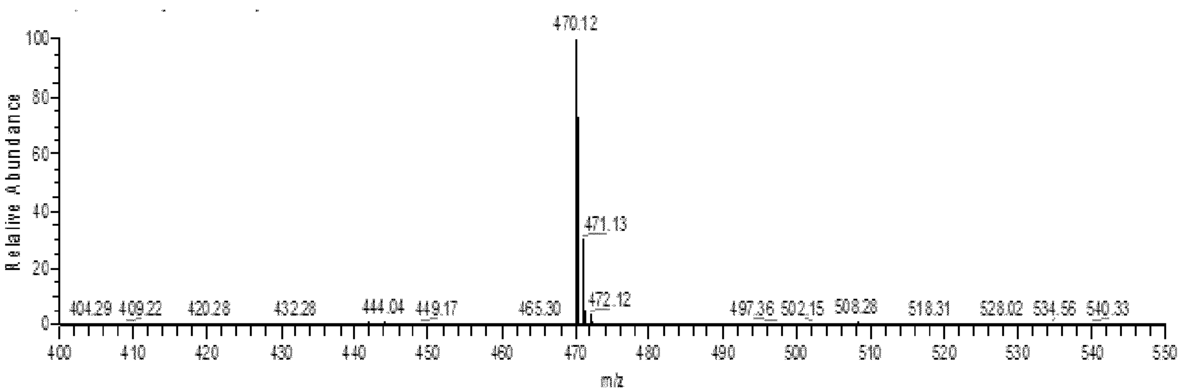
470.12
471.13
472.12
404.29
409.22
420.28
432.28
444.04
449.17
465.30
497.36
502.15
508.28
518.31
528.02
534.56
540.33
Relative Abundance
m/z

Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 103086975 A